# EUROPÄISCHE PATENTSCHRIFT

(11) **EP 4 181 968 B1**
(45) Veröffentlichungstag und Bekanntmachung des Hinweises auf die Patenterteilung: **22.05.2024**
(21) Anmeldenummer: 21740238.7
(22) Anmeldetag: 14.07.2021
(51) Int. Cl.: A61L 2/10, A61L 2/00

(54) **VORRICHTUNG UND VERFAHREN ZUR VERMINDERUNG ODER BESEITIGUNG VON KRANKHEITSERREGERN**
DEVICE AND METHOD FOR REDUCING OR ELIMINATING PATHOGENS
DISPOSITIF ET PROCÉDÉ DE RÉDUCTION OU D'ÉLIMINATION D'AGENTS PATHOGÈNES

(30) Priorität: 15.07.2020 CH 8792020; 23.10.2020 CH 13672020
(43) Veröffentlichungstag der Anmeldung: 24.05.2023
(73) Patentinhaber: JK-Holding GmbH, 53578 Windhagen (DE)
(72) Erfinder: GERSTENMEIER, Jürgen, 56567 Neuwied (DE)
(74) Vertreter: IPrime Rentsch Kaelin AG
(86) Internationale Anmeldenummer: PCT/IB2021/056341
(87) Internationale Veröffentlichungsnummer: WO 2022/013771

(56) Entgegenhaltungen:
- WO-A1-2016/064441
- WO-A1-2016/196904
- US-A1- 2019 255 201
- US-A1- 2020 215 210

## Beschreibung

Die vorliegende Erfindung betrifft eine Vorrichtung und ein Verfahren zur Verminderung oder Beseitigung von Krankheitserregern.

Weiter betrifft die Erfindung die Verwendung eines Leuchtmittels in derartigen Vorrichtungen zur Reduzierung und Beseitigung von Krankheitserregern sowie ein Verfahren alles jeweils gemäss den Oberbegriffen der unabhängigen Ansprüche.

### Technologischer Hintergrund

Krankheitserreger verursachen im menschlichen oder tierischen Körper Abläufe, die gesundheitsschädigend sind. Meistens handelt es sich bei den Krankheitserregern selbst um Mikroorganismen, wie etwa Bakterien, Pilze, Parasiten oder Algen. Viren hingegen sind infektiöse Partikel, die sich nur mithilfe des Wirts vermehren können; bei pathogenen Prionen handelt es sich um Proteine und damit um organische Gifte. Krankheitserreger können heftige Reaktionen des Immunsystems auslösen, die im schlimmsten Fall zum Tod führen können. Je nach Gefährlichkeit, Ansteckungsrisiko und Verbreitung werden diese in verschiedene Gruppen eingeteilt.

Im Zuge der Verbreitung von SARS-CoV-2 hat das öffentliche Leben zahlreiche Einschränkungen erfahren. Es zeichnet sich vermehrt ab, dass einige dieser Einschränkungen insbesondere bezüglich des Verhaltens im öffentlichen Raum und des Zusammenlebens auch nachhaltig sein werden. Eine Lehre aus der Covid-19-Pandemie ist zweifellos, dass in einer hypermobilen Gesellschaft und hoher Bevölkerungsdichte in Ballungszentren eine Verbreitung von Seuchen nur schwer global zu verhindern ist. Zahlreiche gesellschaftliche Massnahmen dienen dazu, die Infektionsrisiken im öffentlichen Raum zu senken. Nicht zuletzt leidet aber das Vertrauen der Menschen in öffentlich zugängliche Vorrichtungen, Gegenstände oder Bereiche, die mitunter ein gewisses Übertragungsrisiko mit sich bringen können.

Kurzfristig können auf personeller Seite Massnahmen ergriffen werden, um stets hygienisch einwandfreie Zustände in öffentlich benutzbaren Vorrichtungen zu garantieren. Letztlich führt dies allerdings zu höheren Kosten im Betrieb und Unterhalt solcher Vorrichtungen. Besonders betroffen können öffentlich zugängliche Anlagen sein, an denen Keime, Bakterien, oder Viren anzutreffen sind, wie z.B. Sport- und Massagegeräte oder Entspannungsliegen im öffentlichen Raum. Gerade in Bereichen, in denen ein hoher Publikumsverkehr herrscht, lässt sich ohne übermässigen Aufwand kaum eine dauerhafte hygienisch einwandfreie Benutzeroberfläche von Geräten garantieren. Besonders betroffen und kritisch sind in diesem Zusammenhang z.B. Flughäfen, Wartezonen, Innenstadtbereiche und Einkaufszentren.

Aber auch im häuslichen oder beruflichen Bereich besteht vermehrt das Bedürfnis nach hygienisch einwandfreien Bereichen und Oberflächen.

Im gängigen Gebrauch werden vorwiegend Desinfektionsmittel in flüssiger Form oder Tücher mit chemischen Mitteln eingesetzt, die allerdings zu Nebenwirkungen oder Hautreizungen führen können.

Stets muss ein Nachschub an geeigneten Desinfektionsmitteln bereitstehen. Viele Desinfektionsmittel sind zudem aggressiv und können bei Gebrauch die Haut austrocknen oder sogar schädigen. Weiter sieht das Verwenden eines Desinfektionsmittels die Manipulation eines weiteren Gegenstandes vor, was wiederum zu einem möglichen Kontaminationsrisiko führen kann. Keime können resistent werden.

Es ist bekannt, dass ultraviolette Strahlung zur Desinfektion verwendet werden kann (siehe Dokument WO2016/064441 A1).

Dazu wird die zu desinfizierende Oberfläche und/oder das zu desinfizierende Fluid mit UV-Strahlung beaufschlagt, welche Mikroorganismen zerstört und inaktiviert. Gängigerweise wird Strahlung in einer Wellenlänge von 254 nm verwendet. Sollen zusätzlich noch organische Verbindungen aufgespalten werden, so werden z.B. Wellenlängen von kleiner als 200 nm verwendet. Solche Wellenlängen sind bekannterweise schädlich für Menschen. Aus diesem Grund sind Leuchtmittel mit den genannten Wellenlängenbereichen in der Regel in nicht zugänglichen Bereichen, z.B. in Lüftungen oder in Filtern, um sicherzustellen, dass ein Kontakt der Haut mit der schädlichen UV-Strahlung möglichst verhindert wird.

Es besteht somit ein Bedarf nach Desinfektionsmitteln, welche für Menschen sicher und mit möglichst wenig Nebenwirkungen verwendbar sind. Weiter sollen diese Desinfektionsmittel vorzugsweise bewegbar dort eingesetzt werden können, wo Bedarf ist.

### Darstellung der Erfindung

Es ist somit eine Aufgabe der vorliegenden Erfindung, eine Vorrichtung eingangs erwähnter Art bereitzustellen, die mindestens einen Nachteil des Bekannten überwindet. Insbesondere soll eine tragbare Vorrichtung bereitgestellt werden, die eine hohe Akzeptanz bei den Benutzern hinsichtlich der hygienischen Reinheit aufweist. Dabei soll die genannte Vorrichtung vorzugsweise möglichst effizient im Betrieb sein und keine weiteren Personalkosten mit sich bringen.

Besonders bevorzugt ist die erfindungsgemässe Lösung, um den hygienischen Anforderungen des 21. Jahrhunderts gerecht zu werden.

Mindestens eine dieser Aufgaben wurde mit einer Vorrichtung zur Verminderung oder Beseitigung von Krankheitserregern gemäss den Merkmalen der unabhängigen Ansprüche gelöst.

Ein Aspekt der vorliegenden Erfindung betrifft eine tragbare Vorrichtung zur Verminderung oder Beseitigung von Krankheitserregern. Die Vorrichtung kann auch als Desinfektion-Strahler Verwendung finden. Die Vorrichtung kann zur Verminderung oder Beseitigung von Krankheitserregern auf Oberflächen oder für Bereiche eingesetzt werden. Dies gilt z.B. für Geräte, Gegenstände, Sachen, Textilien, Stoffe oder auch Umgebungsbereiche, die potentielle Krankheitserreger aufweisen, aber auch für Lebens- oder Nahrungsmittel und sogar für Wasser und andere Flüssigkeiten.

Die erfindungsgemässe Vorrichtung umfasst eine Anordnung gemäss unabhängigen Anspruch 1.

Im Sinne der vorliegenden Erfindung bedeutet die Steuerung des Leuchtmittels das Einund Ausschalten sowie die Anpassung der Leistung oder Intensität entsprechend der ermittelten Parameter.

Besonders bevorzugt ist der Strahlungsbereich so ausrichtbar, dass ein Objekt oder eine Oberfläche im Wesentlichen vollständig beaufschlagbar ist, insbesondere so dass diese stets einer optischen Strahlung im genannten Wellenlängenbereich ausgesetzt ist. Wird die Vorrichtung vor einen Benutzer gestellt oder positioniert, so kann beispielsweise die optische Strahlung auch den Benutzer beaufschlagen.

Es wurde überraschend gefunden, dass im besagten Wellenlängenbereich keine ansonsten von UVC-Strahlung erwartete Schädigung der menschlichen Haut eintritt (Longterm effects of 222 nm ultraviolet radiation C sterilizing lamps on mice susceptible to ultraviolet radiation, Yamano, Nozomi et al., Photochemistry and Photobiology, doi: 10.1111/php.13269).

Als besonderer Vorteil der vorliegenden Erfindung werden nicht nur Objekte oder Oberflächen, sondern auch ein im Strahlungsbereich befindliches Fluid, z.B. Luft oder Wasser mit der desinfizierenden Strahlung beauftragt. Da die besagte Strahlung im erfindungsgemässen Bereich keine ansonsten von UVC-Strahlung erwartete Schädigung der menschlichen Haut auslöst, kann somit eine besonders sichere Umgebung für einen Nutzer sichergestellt werden, was einem erhöhtem Sicherheitsbedürfnis besonders in Zeiten erhöhter pandemischer Alarmbereitschaft entgegen kommt.

Die erfindungsgemässe Vorrichtung kann für Menschen, Tiere, Gegenstände, Geräte, Bereiche und Flächen verwendet werden, um Krankheitserreger zu vermindern oder gar zu beseitigen. Diese können unabhängig von der Gewissenhaftigkeit eines reinigenden Mitarbeiters stets in einen hygienisch unbedenklichen Zustand gebracht werden. Die besagte UV-Strahlung im genannten Bereich ist zudem vorzugsweise so gewählt, dass stets mindestens alle in der Nutzung verwendeten Oberflächen oder Flächen mit der besagten UV-Strahlung beaufschlagbar sind. Im Betrieb kann die UV-Lampe mit der entsprechenden Strahlung ausgeschaltet sein oder weiterlaufen. Bei Letzterem findet zudem eine Desinfektion der entsprechenden Oberfläche statt, was zusätzlich ein gewünschter Effekt sein kann. Zudem wird auch der Strahlungsbereich, welcher als ein Luftraum zwischen einer Fläche oder einem Objekt und dem Leuchtmittel definiert sein kann, mit der genannten Strahlung beaufschlagt, und desinfiziert diesen.

In einer besonderen Ausführungsform ist das Leuchtmittel ausgelegt, im Wesentlichen monoenergetische UV-Strahlung mit einem Peak einer Wellenlänge im Bereich von zwischen 207 und 222 nm abzustrahlen und mit einer Energie im Strahlungsbereich von zwischen 0.5 mJ/cm² bis 500 mJ/cm², insbesondere von zwischen 2 mJ/cm² und 50 mJ/cm², ganz besonders bevorzugt von ca. zwischen 2 mJ/cm² und 20 mJ/cm².

Die Energie kann zum Beispiel so definiert sein, dass sie als Dosis innerhalb des Strahlungsbereichs zu verstehen ist, wobei der Strahlungsbereich ein Volumen definiert, eingeschlossen von den Abstrahlwinkeln des Leuchtmittels. Besonders bevorzugt ist das Leuchtmittel so ausgelegt, dass die genannte Energie im Strahlungsbereich in einem Abstrahlwinkel mit Schenkellängen von zwischen 0m und 2 m, insbesondere zwischen 0.5 m und 2 m auftritt. Der Abstrahlwinkel kann insbesondere dann eine Schenkellänge von 0 haben, wenn die Vorrichtung ausgelegt ist durch Kontakt den zu erzielenden Desinfektionseffekt zu erreichen. So kann zum Beispiel die Vorrichtung ausgelegt sein, um auf eine zu desinfizierende Oberfläche oder ein zu desinfizierendes Objekt gelegt zu werden.

In einer besonderen Ausführungsform ist das Leuchtmittel ausgelegt, um eine einstellbare Energie von im Wesentlichen monoenergetischer UV-Strahlung mit einem Peak einer Wellenlänge im Bereich von zwischen 207 und 222 nm abzustrahlen. Besonders bevorzugt ist das Leuchtmittel ausgelegt über eine Steuerung eine besagte Energie in einem Strahlungsbereich mit besagter optischer Strahlung bereitzustellen. Die Energie kann zum Beispiel von einem Fachmann anhand der Geometrie der Vorrichtung, des Objekts und/oder des Strahlungsbereichs einstellbar sein. Die Energie kann auch insbesondere aufgrund eines bestimmten zu bekämpfenden Keims einstellbar sein, wobei die Steuerung bevorzugt so ausgestaltet ist, dass ein eingestellter Keim oder Krankheitserreger eine besondere Einstellung der Energie des Leuchtmittels für den besagten Strahlungsbereich auslöst.

Besonders ist das Leuchtmittel ausgestaltet, um Bakterien aus der Gattung Haemophilus zu inaktivieren mit UV-Strahlung mit einem Peak in einem Wellenlängenbereich von zwischen 207 und 222 nm und mit einer Energie von zwischen mindestens 0.5 mJ/cm² bis höchstens 10 mJ/cm² im Strahlungsbereich.

Besonders ist das Leuchtmittel ausgestaltet, um Viren aus der Familie der Coronaviren zu inaktivieren mit UV-Strahlung mit einem Peak in einem Wellenlängenbereich von zwischen 207 und 222 nm und mit einer Energie von zwischen mindestens 0.3 mJ/cm2 bis 2 mJ/cm2 im Strahlungsbereich.

Besonders ist das Leuchtmittel ausgestaltet, um Viren aus der Familie der Influenzaviren zu inaktivieren mit UV-Strahlung mit einem Peak in einem Wellenlängenbereich von zwischen 207 und 222 nm und mit einer Energie von zwischen mindestens 0.4 mJ/cm² bis 6 mJ/cm² im Strahlungsbereich.

Besonders bevorzugt ist das Leuchtmittel ausgelegt, im Wesentlichen monoenergetische UVC-Strahlung mit einem Peak einer Wellenlänge von 222 nm abzustrahlen.

Es wurde überraschend gefunden, dass mit den genannten Energien und dem entsprechenden Wellenlängenbereich eine hohe Zuverlässigkeit an Desinfektion der beaufschlagten Oberfläche erreichbar ist, und gleichzeitig die Vorteile der entsprechenden Unschädlichkeit der UVC-Strahlung im genannten Wellenlängenbereich erreichbar sind.

Ohne an diese Theorie gebunden zu sein, so scheint es sich bei den genannten Wellenlängenbereichen um Wellenlängen zu handeln, die vorwiegend in der Hautoberfläche, der Cuticula, aufgenommen werden und denen es nicht gelingt, in menschliche Zellen einzudringen und dort entsprechend den nicht gewünschten Zellschaden anzurichten, wie ihn anderweitige UV-Strahlung anzurichten vermag.

In einer besonderen Ausführungsform umfasst das Leuchtmittel eine Excimer-basierende Lampe. Insbesondere umfasst das Leuchtmittel eine quasi monochromatische Lichtquelle. Alternativ und/oder ergänzend umfasst das Leuchtmittel mindestens einen Kurzpass- und/oder Bandpassfilter zur Reduktion eines Emissionsspektrums des Leuchtmittels auf eine Wellenlänge mit einem Peak im genannten Bereich, insbesondere bei ca. 207 nm oder 222 nm.

Besonders bevorzugt umfasst das Leuchtmittel eine Lampe mit den Excimer-Molekülen, ausgewählt aus der Gruppe, bestehend aus: Krypton-Chlor oder Krypton-Brom (Kr-Cl, Kr-Br).

In einer besonderen Ausführungsform umfasst das Leuchtmittel einen Kurzpassund/oder Bandpassfilter, der ausgelegt ist, Wellenlängen ausserhalb einer Wellenlänge im Bereich von zwischen 207 nm und 230 nm zu entfernen. Mit anderen Worten, der Bandpassfilter ist so ausgestaltet, dass er Wellenlängen von kürzer als 226 nm im Wesentlichen hindurchlässt. Im Sinne der vorliegenden Erfindung sei hindurchlässt verstanden, als eine optische Durchlässigkeit von wenigstens 60%, bevorzugt wenigstens 80%, besonders bevorzugt wenigstens 90% aufweisend für optische Strahlung der betreffenden Wellenlänge.

In einer weiteren besonderen Ausführungsform umfasst das Leuchtmittel einen Kurzpassfilter mit einer optischen Durchlässigkeit von wenigstens 60%, bevorzugt wenigstens 80% für Wellenlängen, die kürzer als 230 nm sind. Besonders bevorzugt weist der Kurzpassfilter eine Kante in einem Bereich von zwischen 226 und 232 nm auf. In einer weiteren besonderen Ausführungsform weist der Kurzpassfilter einen Interferenzfilter aus mindestens einer, vorzugsweise zweier Filterschichten auf.

In einer besonders bevorzugten Ausführungsform umfasst das Leuchtmittel eine Excimer-basierende Lampe, die im Wesentlichen Licht einer Wellenlänge mit einem Peak von 207 nm emittiert, insbesondere einer Wellenlänge mit einem Peak von im Wesentlichen 207 nm bei der bei einer relativen Leistung von 10% oder mehr das Emissionsspektrum grösser als 200 nm und kleiner als 214 nm ist, besonders bevorzugt grösser als 204 nm und kleiner als 210 nm ist.

In einer alternativen besonderen Ausführungsform umfasst das Leuchtmittel eine Excimer-basierende Lampe, welche im Wesentlichen Licht einer Wellenlänge mit einem Peak von von 222 nm emittiert, insbesondere einer Wellenlänge mit einem Peak von im Wesentlichen 222 nm bei der bei einer relativen Leistung von 10% oder mehr das Emissionsspektrum grösser als 215 nm und kleiner als 229 nm ist, besonders bevorzugt grösser als 219 nm und kleiner als 225 nm ist.

Um den emittierten Wellenlängenbereich zu erreichen, kann ein entsprechendes Dimer-Paar, z.B. Krypton-Chlor oder Krypton-Brom verwendet werden und in einer besonderen Ausführungsform zusätzlich ein geeigneter Kurzpass- und/oder Bandpassfilter vorgesehen sein.

In einer besonderen Ausführungsform umfasst das erfindungsgemässe Leuchtmittel eine Kühlung. Besonders bevorzugt is t einen Strömungserzeuger am Leuchtmittel vorgesehen, um Kühlung durch Luft zu erzeugen.

In einer besonderen Ausführungsform kann eine Wärmeleiterstruktur vorgesehen sein, um einen Wärmeaustausch zwischen Lampe und Umgebung zu erleichtern, so können insbesondere oberflächenerweiternde Lamellen vorgesehen sein, welche die Abwärme ableiten. Zusätzlich oder alternativ können Strömungserzeuger wie Ventilatoren verbaut werden, welche eine Ansammlung von durch die Abwärme des Lampenbetriebs entstehende Hitze abführen können.

In einer besonderen Ausführungsform umfasst die Vorrichtung eine Mehrzahl an Leuchtmitteln mit jeweils einem Strahlungsbereich.

In einer besonderen Ausführungsform ist der Strahlungsbereich so ausgelegt, dass mindestens ein Objekt oder eine Oberfläche im Wesentlichen vollständig erfasst und beaufschlagbar wird mit optischer Strahlung mit einem Peak im Bereich von zwischen 207 und 222 nm. Zusätzlich oder alternativ kann der Strahlungsbereich so gewählt oder von der Steuereinheit eingestellt werden, dass in Benutzung zu den Objekten oder Oberflächen zusätzlich noch angrenzende Flächen und Bereiche beaufschlagt werden.

In einer besonderen Ausführungsform ist das Leuchtmittel ausgelegt, optische Strahlung mit einem Peak im Bereich ca. 207 oder ca. 222 nm zu emittieren mit einer Halbwertsbreite von ca. 4 nm.

In einer besonderen Ausführungsform umfasst die erfindungsgemässe Vorrichtung eine Steuereinheit, die vorzugsweise in der Griffeinheit angeordnet ist. Die Steuereinheit kann auch in der Nähe der Bedienelemente angeordnet sein, was eine verkürzte Leitungsführung ermöglicht.

Die Griffeinheit kann so ausgestaltet sein, dass für den Betrieb der Vorrichtung bzw. des Leuchtmittels beide Hände die Griffeinheit berühren müssen (Zweihand- oder Beidhandbetrieb). So kann eine sachgerechte und sichere Handhabung der Vorrichtung garantiert werden.

Die Steuereinheit kann z.B. Strahlungsintensitäten, Intervalle, oder die geometrische Ausrichtung des Strahlungsbereichs jeweils an entsprechende Gegebenheiten anpassen. So kann die Steuereinheit z.B. ausgestaltet sein, um entsprechende Programme oder ein Wartungsprogramm durchzuführen.

Das Bedienelement kann vorteilhafterweise ein Display oder Touchscreen enthalten, welches dem Benutzer Instruktionen zur Handhabung anzeigt oder eine Benutzerführung angibt. Die Benutzerführung kann interaktiv zum Beispiel in Verbindung mit einer Objekterkennung oder einer Geräteerkennung erfolgen.

Ebenfalls kann die Steuereinheit ausgestaltet sein, um eine entsprechende Neuorientierung der Leuchtmittel vorzunehmen, wenn z.B. die Vorrichtung benutzt wird. Besonders bevorzugt ist die Steuereinheit so ausgestaltet, dass durch die Steuereinheit eine Energie mJ/cm² im Strahlungsbereich einstellt wird. Dies kann insbesondere durch die Erfassung der Umgebung durch die Detektiereinheit erfolgen. Wenn beispielsweise Haut, ein Gesicht oder Augen erkannt werden, wird die Steuereinheit die Energie im Strahlungsbereich entsprechend anpassen, d.h. reduzieren oder abschalten.

Die mindestens eine Detektiereinheit dient der Ermittlung von Parametern. Verschiedenste Detektoren können zum Einsatz kommen.

In einer besonderen Ausführungsform umfasst die erfindungsgemässe Vorrichtung Detektoren oder Sensoren, welche z.B. das Benutzen der Vorrichtung detektieren. Weiter können z.B. Sensoren vorgesehen sein, welche Masse oder Temperaturen erfassen. Die Steuereinheit kann dann so ausgelegt sein, um die entsprechende Beaufschlagung der Flächen an die Oberflächengegebenheiten oder an die Detektion eines Menschen anzupassen.

In einer besonderen Ausführungsform umfassen die erfindungsgemässen Leuchtmittel einen Kontaktschutz, welcher verhindert, dass die Leuchtmittel von den benutzenden Menschen berührt werden können. So können insgesamt die Lebensdauer der Leuchtmittel erhöht und eine Gefahr von Verbrennung minimiert werden. In der einfachsten Ausführung kann z.B. ein Gitter oder eine Glasscheibe vorgesehen sein, welche verhindert, dass das Leuchtmittel berührt wird.

Da Menschen nicht in der Lage sind, Licht in den genannten Spektren wahrzunehmen, ist der Einsatz der erfindungsgemässen Vorrichtung mit zusätzlichen Leuchtmitteln mit sichtbarem Licht oder Rastern kombinierbar. Zudem ist mittels Displays die Darstellung des Strahlungsbereichs möglich.

Die Strahlung des Leuchtmittels beaufschlagt nicht nur Objekte oder Flächen, sondern auch Bereiche, die sich im Strahlungsbereich befinden, wie Luft oder ein Fluid wie Wasser o.ä.

In einer besonderen Ausführungsform ermittelt die Detektiereinheit den Abstand zu einer Oberfläche oder einem Objekt. Dadurch kann die entsprechend notwendige Intensität eingestellt bzw. bereitgestellt werden. Die Steuereinheit kann die Intensität in Echtzeit anpassen, wenn sich zum Beispiel der Abstand oder die Lage ändert.

In einer besonderen Ausführungsform umfasst die Detektiereinheit einen Sensor, der insbesondere ausgelegt ist, um die Lage des Leuchtmittels relativ zu einem Objekt oder einer Oberfläche zu erfassen. Ein geeigneter Sensor wäre z.B. ein Infrarot- oder Distanzsensor. Ebenfalls denkbar ist es, einen Accelerometer oder ein Magnetometer vorzusehen, welches die Ausrichtung des Leuchtmittels im Raum zu erfassen vermag. Im Zusammenspiel mit der Steuereinheit kann so z.B. ein entsprechend der Vorrichtung angepasstes Programm durchgeführt werden. Wenn die Detektiereinheit die Lage des Leuchtmittels relativ zu einem Objekt oder einer Oberfläche erfassen kann, ist die mögliche Strahlungsrichtung bekannt. Je nach Richtung kann die Intensität entsprechend angepasst werden. Aus Sicherheitsgründen kann es notwendig sein, dass das Leuchtmittel in einer Lage möglicherweise keine oder nur eine geringe Strahlung abgibt, wohingegen in einer anderen Lage die volle Leistung abgegeben werden kann.

In einer zusätzlichen und/oder alternativen Ausführungsform ist die Detektiereinheit ausgebildet, um eine Benutzung zu detektieren und entsprechend einen Abstrahlwinkel anzupassen, der somit den Strahlungsbereich definiert.

Gemäss der Erfindung führt die Detektiereinheit eine optische Erkennung durch. Dafür ist die Detektiereinheit mit optischen Sensoren ausgestattet. Mittels Bilderkennung kann erkannt werden, um welchen Bestrahlungsgegenstand es sich zum Beispiel handelt. Bei einem Gegenstand oder einer Oberfläche kann die Intensität der Strahlung sicher höher sein oder länger einwirken als bei lebenden Objekten. Ja nach erkanntem Objekt oder Gerät können Programme mit verschiedenen, z.B. Intensitäten und Längen aktiviert werden.

Sobald die Detektiereinheit eine Bewegung ermittelt, ist klar, dass die Vorrichtung nicht stillgehalten wird. In einer besonderen Ausführungsform werden über das Display des Bedienelements Anweisungen an den Benutzer ausgegeben, z.B. Bewegungsrichtung, Bewegungsgeschwindigkeit, oder Abstand. Diese können dann entsprechend variiert werden, um ein optimales Bestrahlungsergebnis zur Verminderung oder Beseitigung von Krankheitserregern zu erzielen.

Gemäss der Erfindung werden mittels Detektiereinheit Objekte erkannt. Das hat den Vorteil, dass vorgespeicherte Programme, die Objekten zugeordnet sind, einfach abgerufen werden können. Objekte können Geräte sein, die beispielsweise von verschiedenen Benutzern benutzt werden und somit regelmässig in einen hygienisch einwandfreien Zustand zu bringen sind. An den Objekten oder Geräten kann sich ein Barcode befinden, der von der Vorrichtung mittels Detektiereinheit zur richtigen Strahlungsanwendung eingelesen wird. Möglich ist auch RFID zu verwenden, um Geräte zu erkennen. Je nach Objekt- und Geräteerkennung können durch die Steuereinheit verschiedene oder bereits angepasste Programme zur optimalen Beseitigung von Krankheitserregern verwendet werden. Eine erforderliche Mindestdosis kann automatisch bereitgestellt werden. Erfolgt jedoch eine Gesichtserkennung wird zur Sicherheit eine maximale Dosierung vorgegeben oder die Dosis entsprechend reduziert. Sollten z.B. Augen erkannt werden, kann zum Beispiel die Dosis reduziert werden aus Sicherheitsgründen, um die Augen zu schützen.

Die Detektiereinheit kann auch eine Kontrolle der Desinfektion ermöglichen, indem bestimmte ermittelte Werte mit Referenzwerten verglichen werden.

In einer besonderen Ausführungsform umfasst die erfindungsgemässe Vorrichtung weiter eine Lüftung zur Erzeugung einer Lüftungsströmung oder Kühlung. In der einfachsten Ausführungsform kann z.B. ein Strömungserzeuger oder Ventilator vorgesehen sein, welcher Luft dem Strahlungsbereich und/oder dem Leuchtmittel zuführt.

In einer besonderen Ausführungsform ist das Leuchtmittel hinter einer Kontaktfläche angeordnet, sodass die Kontaktfläche von hinten, d.h. durch das Flächenmaterial hindurch, beaufschlagt wird. Für diese Ausführungsform ist es erforderlich, dass die Kontaktfläche für die bezeichnende Strahlung im Wellenlängenbereich von zwischen 200 und 230 nm durchlässig ist.

Mit der erfindungsgemässen Vorrichtung ist sichergestellt, dass in Zeiten erhöhter pandemischer Alarmbereitschaft das Vertrauen von Benutzern gewahrt bleibt. Die erfindungsgemässe Vorrichtung erlaubt stets Objekte, Oberflächen, oder Bereiche in einem hygienisch einwandfreien Zustand zu halten. Zusätzliche hautreizende Mittel entfallen.

Ein weiterer Aspekt der vorliegenden Erfindung betrifft eine Verwendung mindestens eines Leuchtmittels, das ausgelegt ist, optische Strahlung in einem Wellenlängenbereich von zwischen 200 nm und 300 nm abzustrahlen zur Erzeugung eines Strahlungsbereichs mit optischer Strahlung mit einem Peak in einem Wellenlängenbereich von zwischen 207 nm und 222 nm in einer tragbaren Vorrichtung.

In einer besonderen Ausführungsform umfasst die erfindungsgemässe Verwendung zudem das Verbinden von Schnittstellen, wobei das Leuchtmittel entsprechende Schnittstellen zur Steuereinheit aufweist. Steuerfunktion werden von der Steuereinheit bereitgestellt, welche von dem Bedienelement oder der Bedieneinheit und der Detektiereinheit beeinflusst werden.

Ein weiterer Aspekt der vorliegenden Erfindung betrifft ein Verfahren zur Verminderung oder Beseitigung von Krankheitserregern.

Das Verfahren umfasst die Schritte des Bereitstellens einer erfindungsgemässen Vorrichtung, das Positionieren der Vorrichtung, so dass mindestens ein zu behandelnder Bereich sich im Strahlungsbereich befindet, und das Beaufschlagen des Strahlenbereichs mit Strahlung mit einem Peak in einem Wellenlängenbereich von zwischen 207 nm und 222 nm.

In einer besonderen Ausführungsform umfasst das erfindungsgemässe Verfahren, dass das Positionieren durch das Bedienelement von der Steuereinheit aufgrund einer erfassten Lage des Leuchtmittels unterstützt wird.

Das Leuchtmittel ist ausgelegt, um optische Strahlen mit einem Peak in einem Wellenlängenbereich von zwischen 207 nm und 222 nm abzustrahlen. Dazu verfügt das Leuchtmittel über einen Abstrahlwinkel, der den Strahlungsbereich definiert. In diesem Strahlungsbereich wird alles mit Licht mit einem Peak in einem Wellenlängenbereich von zwischen 207 nm und 222 nm beaufschlagt. Der Strahlungsbereich wird mit Strahlung in der genannten Wellenlänge beaufschlagt.

In einer besonderen Ausführungsform umfasst das erfindungsgemässe Verfahren ein Filtern optischer Strahlung in einem Wellenlängenbereich von zwischen 200 nm und 230 nm, so dass ein Strahlungsbereich mit optischer Strahlung mit einem Peak in einem Wellenlängenbereich von zwischen 207 nm und 222 nm, insbesondere von 207 nm oder 222 nm beaufschlagbar ist. Besonders bevorzugt umfasst das Filtern das Bereitstellen eines Kurzpassfilters und/oder Bandpassfilters, insbesondere eines Kurzpassfilters mit einer Kante in einem Wellenlängenbereich von zwischen 226 nm und 232 nm.

In einer besonderen Ausführungsform findet das Positionieren mittels Hand statt, wird jedoch von der Steuereinheit und dem Bedienelement unterstützt. Die Steuereinheit führt anhand von bekannten oder detektierten Werten und/oder Daten eine Beaufschlagung durch. Die Beaufschlagung kann in diesem Fall z.B. an die geometrischen Besonderheiten der Umgebung angepasst sein. Dazu kann z.B. das Leuchtmittel dergestalt bewegt werden, dass der Strahlungsbereich verschiedene Flächen unterschiedlich lange beaufschlägt.

In einer weiteren Ausführungsform umfasst das erfindungsgemässe Verfahren das Aufzeichnen der Benutzung der Vorrichtung über einen Zeitraum. Der Zeitraum kann dabei die Nutzugsdauer der Vorrichtung sein, kann jedoch Intervalle oder manuell einstellbare Zeiten umfassen. Eine Protokollierung der Nutzung erlaubt eine spätere Auswertung. Bei erfolgreicher Nutzung können Werte, die sich als effektive ergeben haben, ausgelesen, übertragen und anderen Orts eingesetzt werden.

Im Folgenden wird nun die vorliegende Erfindung anhand konkreter Ausführungsbeispiele und Figuren näher erläutert, ohne jedoch auf diese beschränkt zu sein. Für einen Fachmann ergeben sich aus dem Studium dieser konkreten Ausführungsbeispiele weitere vorteilhafte Ausführungsformen. Der Einfachheit halber werden in den Figuren die gleichen Teile mit den gleichen Bezugszeichen versehen.

### Figurenbeschrieb

Anhand der nachfolgenden Figuren werden Ausführungsbeispiele der Erfindung beschrieben. Es zeigen
- Fig. 1: eine Ausführung einer erfindungsgemässen Vorrichtung;
- Fig. 2: eine alternative Ausführung einer erfindungsgemässen Vorrichtung;
- Fig. 3: eine weitere alternative Ausführung einer erfindungsgemässen Vorrichtung;
- Fig. 4: eine Ansicht einer erfindungsgemässen Vorrichtung im Einsatz;
- Fig.5: eine weitere Ansicht einer erfindungsgemässen Vorrichtung im Einsatz;
- Fig. 6: Transmissionskurve eines geeigneten Bandpassfilters, und
- Fig. 7: Ausführungsform eines erfindungsgemässen Wellenlängenbereichs.

### Ausführung der Erfindung

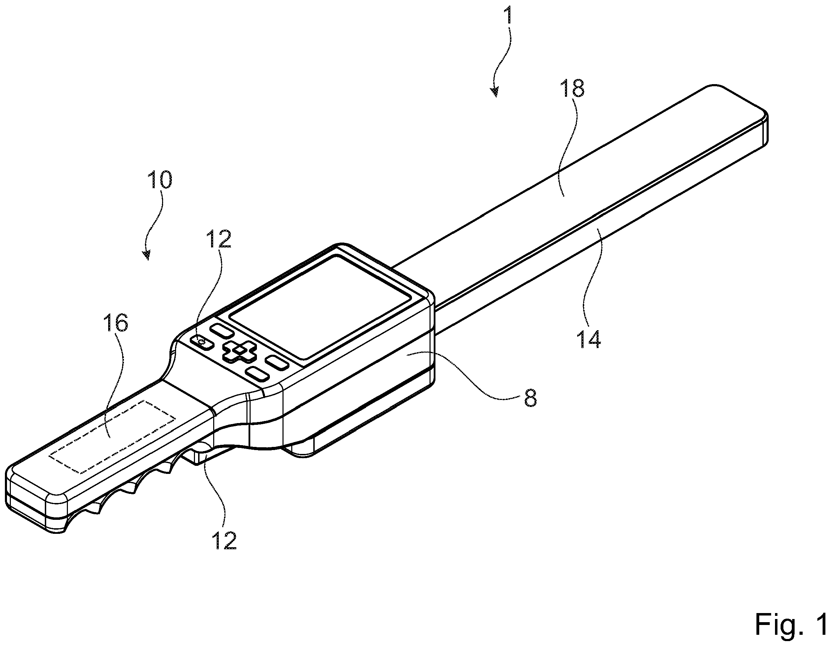

In der **Fig. 1** ist eine grundsätzliche Ausführung einer erfindungsgemässen Vorrichtung gezeigt. Die tragbare Vorrichtung 1 dient der Verminderung oder Beseitigung von Krankheitserregern. Die Vorrichtung 1 weist eine Griffeinheit 10 auf mit mindestens einem Bedienelement 12. Weiter umfasst die Vorrichtung 1 ein Leuchtmittel 18, das mit der Griffeinheit 10 verbindbar und dazu ausgelegt ist, optische Strahlung in einem Wellenlängenbereich von zwischen 200 nm und 230 nm abzustrahlen. Das Leuchtmittel 18 ist ausgelegt, um einen Strahlungsbereich mit optischer Strahlung mit einem Peak in einem Wellenlängenbereich von zwischen 207 nm und 222 nm zu beaufschlagen. Entlang des Leuchtmittels 18, in der Draufsicht dargestellt, ist eine Detektiereinheit 14 zur Ermittlung von Parametern angeordnet. Eine Steuereinheit 16 ist vorzugsweise in der Griffeinheit 10 angeordnet, so dass die Steuereinheit 16 das Leuchtmittel 18 mittels Bedienelement 12 und in Abhängigkeit, der von der Detektiereinheit 14 ermittelten Parameter steuert.

An oder in der Griffeinheit 10 befindet sich eine Energiequelle 8, wie eine Batterie oder ein wiederaufladbarer Akkumulator, der zur Ladung an eine externe Stromquelle angeschlossen werden kann (nicht dargestellt).

Das Leuchtmittel 18 ist im vorliegenden Beispiel so gewählt, dass eine UV-Strahlung mit einer Wellenlänge von 222 nm abgestrahlt wird. Um sicherzustellen, dass die Wellenlänge sich innerhalb eines möglichst schmalen Spektrums mit einem Peak bei 222 nm befindet, wird in diesem Beispiel eine Krypton-Chlor-Excimer-Lampe mit einem schmalen Spektrum verwendet. Zudem wird die Lampe mit einem Bandpassfilter ausgestaltet, der Wellenlängen ausserhalb von 222 nm im Wesentlichen absorbiert. Geeignet sind zum Beispiel Kurzpassfilter aus synthetischem Quarzglas mit ein- oder mehrfacher Beschichtung.

Entsprechende Kurzpass- und/oder Bandpassfilter können den Wellenlängenbereich weiter einschränken, so dass der Peak spezifischer wird und die entsprechenden nachteiligen Effekte der UV-Strahlung auf einen Körper vermieden werden, ohne dass dabei die desinfizierende Wirkung des UV-Lichts geschmälert wird.

Im Betrieb kann das Leuchtmittel 18 ununterbrochen wirken. Allfällige Sicherheitsgrenzwerte und Höchstdosen können eingestellt werden bzw. sind bereits voreingestellt und werden von der Steuereinheit 16 berücksichtigt.

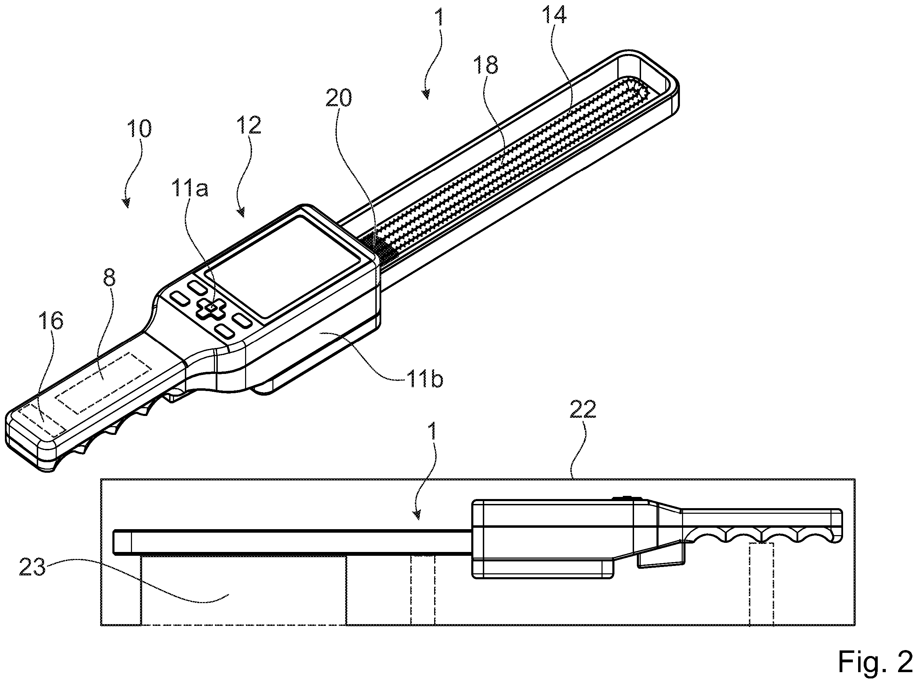

Im vorliegenden Beispiel kann zudem für die Krypton-Chlor-Gaslampe mit einem Peak bei 222 nm eine Luftkühlung 20, wie in Fig. 2, vorgesehen sein. Dazu wird ein Strömungserzeuger oder Ventilator vorgesehen, welcher Luft dem Leuchtmittel zuführt.

In eine weiteren Ausführungsform ist das Leuchtmittel 18 schwenkbar oder klappbar an der Griffeinheit 10 angebracht, sodass eine einfache Verstellbarkeit erreicht werden kann. Wenn die Griffeinheit 10 beispielsweise die gleiche oder eine ähnliche Länge wie das Leuchtmittel 18 aufweist, können diese gegeneinander geklappt werden. Dies dient dem Schutz des Leuchtmittels 18 und einem einfacheren Transport. Die Griffeinheit 10 kann so geformt sein, dass eine mögliche Aufnahme des Leuchtmittels 18 beim Zusammenklappen gewährleistet wird. Alternativ kann eine Schutzhülle oder eine Art Scheide bereitgestellt werden, welche so ausgestaltet ist, dass diese zumindest das Leuchtmittel 18 zum Schutz aufnehmen kann.

In einer besonderen Ausführungsform ist das Leuchtmittel 18 austauschbar. Die Griffeinheit 10 kann somit wiederverwendet werden, falls das Leuchtmittel 18 defekt geht. Möglich ist auch, andere Leuchtmittel zu verwenden, die anderes Licht oder andere Strahlungen abgeben, z.B. im Infrarot-Bereich, zur Wärmebehandlung. Die weiteren Leuchtmittelt sind mit der Griffeinheit 10 kompatibel und werden von der Steuereinheit 16 unterstützt.

Vorzugsweise ist das Leuchtmittel 18 über einen Kontaktschutz davor gesichert, dass es von einem Benutzer berührt werden kann.

In **Fig. 2** weist das Bedienelement 12 ein oder mehre Elemente auf, die zur Inbetriebnahme und Verwendung der Vorrichtung 1 dienen. Ein Schalter 11a ermöglicht beispielsweise das Ein- oder Ausschalten der Vorrichtung 1. Mittels Drehrad 11b kann beispielsweise die Intensität oder Zeitdauer eingestellt werden. Weitere Elemente, die der Steuerung dienen, können angeordnet sein. In der unteren Griffeinheit 10 befindet sich die Energiequelle 8, die die notwendige Energie bereitstellt. Die Steuereinheit 16 ist hier am unteren Ende der Griffeinheit 10 integriert.

Das Leuchtmittel 18 ist an der Griffeinheit 10 drehbar entlang der Längsachse gelagert und ist in Fig. 2 so gedreht, dass optische Strahlung nach oben abgegeben werden kann. Es ist auch möglich, mehrere Leuchtmittel 18 so anzuordnen, dass die optische Strahlung in verschiedene Richtungen abgegeben wird. Die Intensität kann dann je Richtung beeinflusst und gesteuert werden.

In einer bevorzugten Ausführungsform wird eine Aufbewahrungsbox 22 bereitgestellt, in die die Vorrichtung 1 zum Schutz oder zur Ladung der Energiequelle 8 gelegt und transportiert werden kann. Die Box 22 (in Fig.2 etwas verkleinert dargestellt) kann der Kühlung oder Ladung der Vorrichtung 1 dienen. Dafür wird die Box 22 an eine externe Stromquelle angeschlossen (nicht dargestellt). Durch die Box 22 können auch Aktualisierungen von Programmen auf die Steuereinheit 16 übertagen werden. Eine Verbindung zum Internet wird bereitgestellt, entweder von der Box 22 oder alternativ direkt zur Vorrichtung 1.

Die Box 22 kann auch einen Bereich oder eine Aussparung 23 aufweisen, in die Gegenstände zur Beaufschlagung mit der genannten optischen Strahlung gelegt werden können. So können Gegenstände, die häufig in Gebrauch sind, wie z.B. Schlüssel oder Smartphones keimfrei oder nahezu keimfrei werden. Dies kann beispielsweise über Nacht erfolgen, wenn diese Dinge nicht in Gebrauch sind. Eine Ladung des Smartphones mittels der Box 22 kann gleichzeitig vorgenommen werden. Eine Beaufschlagung mit optischer Strahlung kann über einen längeren Zeitraum durchgeführt werden.

Zur Erzeugung einer 207 nm UV-Licht-Wellenlänge kann auch eine Krypton-Brom-Excimer-Lampe verwendet werden, die dann als Leuchtmittel 18 zum Einsatz kommt.

Solche Lampen sind hinsichtlich ihrer Wirkungsweise bekannt (Buonanno M., et al. 207-nm UV Light - A Promising Tool for Safe Low-Cost Reduction of Surgical Site Infections; In Vitro Studies. PLoS One 8(10), 2013).

Die Vorrichtung 1 als Desinfektionsvorrichtung weist ein Leuchtmittel 18 auf, welches eine Excimer-Lampe umfasst, die in der Lage ist, optische Strahlung mit einem Peak in einem Wellenlängenbereich zwischen 207 und 222 nm zu erzeugen. Dabei wird vorzugsweise eine Energie im Strahlungsbereich angepeilt von ca. 2 - 20 mJ/cm².

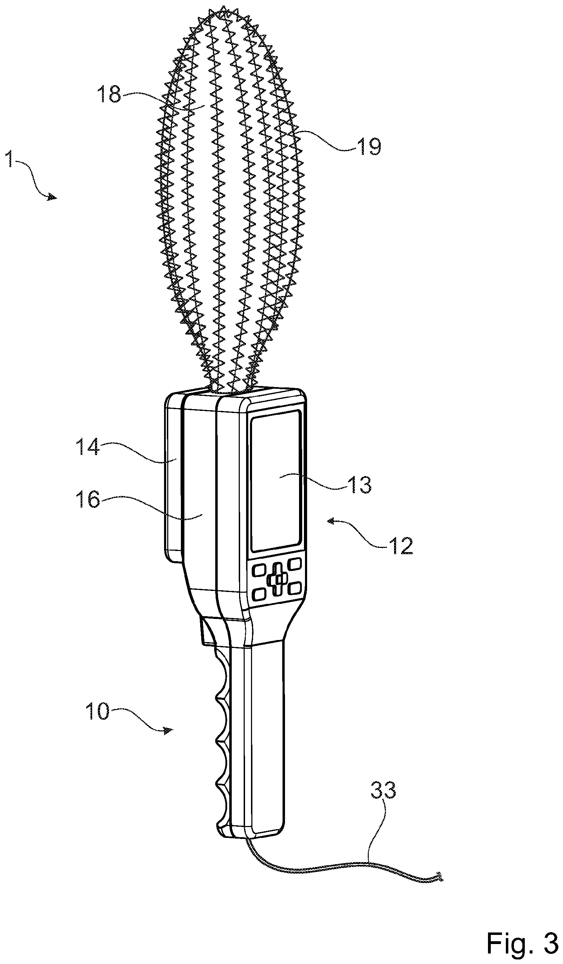

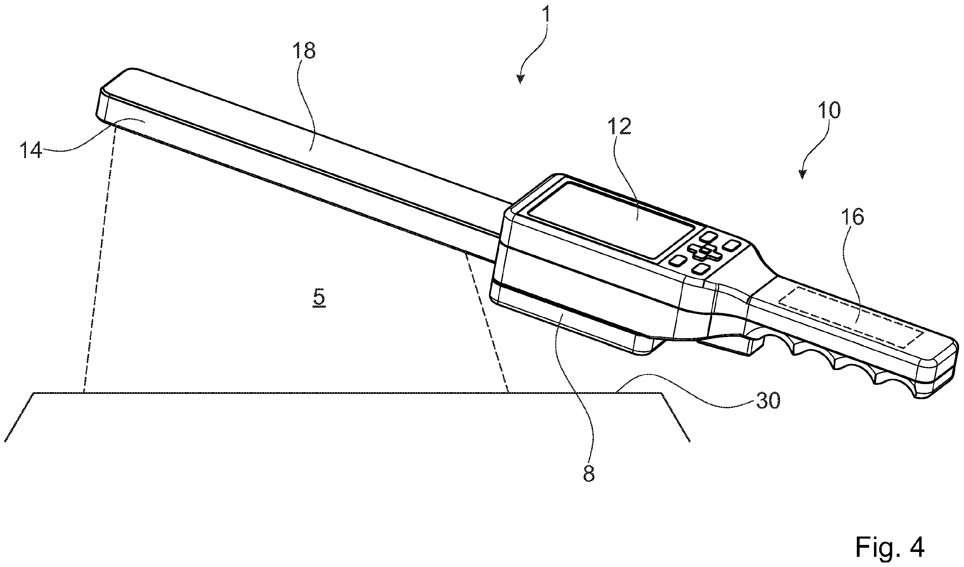

In **Fig. 3** ist die Vorrichtung 1 mit einem Display 13 als Bedienelement 12 ausgestattet. Die in dieser Ausführungsform dargestellte Vorrichtig verfügt über einen direkten Stromanschluss mittels eines Kabels 33. Auf dem Display 13 können ermittelte Parameter angezeigt werden, die von der Detektiereinheit 14 ermittelt wurden. Die Detektiereinheit 14 ist so angebracht und in die Vorrichtung 1 integriert, dass zum Beispiel der Abstand zu einer Oberfläche ermittelt wird, wie in Fig. 4 dargestellt. Die Detektiereinheit 14 kann das Leuchtmittel 18 umgeben, aber auch am Ende der Griffeinheit 10 nahe Display 13 angeordnet sein. Wenn die Detektiereinheit 14 die Lage des Leuchtmittels 18 relativ zu einem Objekt oder einer Oberfläche erfassen kann, ist die mögliche Strahlungsrichtung bereits bekannt und die Intensität kann entsprechend angepasst werden. Aus Sicherheitsgründen kann es notwendig sein, dass das Leuchtmittel 18 in einer Lage möglicherweise keine oder nur eine geringe Strahlung abgibt, wohingegen in einer anderen Lage die volle Leistung abgegeben werden kann.

Wenn die Detektiereinheit 14 eine optische Erkennung durchführt, kann aufgrund der ermittelten Umgebung oder der er- bzw. bekannten Objekte ein voreingestelltes Programm aktiviert werden. Somit kann beispielsweise ein optimiertes Beaufschlagungsprogramm durchgeführt werden. Nach der Bestrahlung sind der bestrahlte Bereich und die Oberfläche keim- oder nahezu keimfrei. Ebenso kann die Detektiereinheit 14 zusammen mit der Steuereinheit 16 eine automatische Abschaltung bewirken, wenn z.B. ein Gesicht oder Augen erkannt werden. Dies dient der Betriebssicherheit und dem Schutz der Augen. Durch die Verwendung von selbstlernenden Programmen passt sich die Vorrichtung schneller an die Benutzung an. Die Steuereinheit 16 ist in dieser Ausführungsform in der Griffeinheit 10 unterhalb der Displays 13 angeordnet.

Sobald die Detektiereinheit 14 eine Bewegung ermittelt, z.B. wenn die Vorrichtung über zu behandelnde Objekte oder Gegenstände hin- und herbewegt wird, kann die Intensität erhöht oder entsprechend eingestellt werden, sodass sich die Desinfektionswirkung verstärkt.

Das Display 13 stellt eine Benutzerführung bereit. Das heisst, ein Benutzer erhält Anweisungen, wie mit der Vorrichtung 1 umgegangen werden muss. Bei der Behandlung von Objekten, Geräten oder Gegenständen zur Verminderung oder Beseitigung von Krankheitserregern wird beispielsweise die optimale Dauer oder der beste Abstand angezeigt. Dieser kann auch variieren, sodass ein Nachführen notwendig sein kann. Ebenfalls können geeignete oder durchzuführende Bewegungen, jedoch auch nützliche Informationen auf dem Display 13 einem Benutzer dargestellt oder angezeigt werden.

Die Handhabung oder Verwendung der tragbaren Vorrichtung 1 kann über einen Zeitraum aufzeichnet werden. Dies lässt Rückschlüsse zu, die der Verbesserung der Vorrichtung 1 bzw. der Programme dienen.

Das Leuchtmittel 18 kann auch in einer Leuchtmittelfassung untergebracht werden und über einen zusätzlichen Reflektorschirm 19 verfügen, welcher einen entsprechenden Abstrahlkegel steuert. Der Reflektor und das Leuchtmittel 18 können in einem Lampengehäuse untergebracht werden. Bevorzugt weist das Lampengehäuse Mittel zur erleichterten Wärmeabfuhr auf, z.B. können Rippen oder Lamellen vorgesehen sein, welche einen einfacheren Wärmeaustausch mit der Umgebungstemperatur ermöglichen.

**Fig. 4** zeigt eine erfindungsgemässe Vorrichtung 1 im Einsatz, d.h. wenn diese in einem Abstand zu einer Oberfläche 30 gehalten wird. Es ergibt sich ein entsprechender Strahlungsbereich S. Dieser Strahlungsbereich S kann als Parameter in die Steuereinheit 16 eingespeist werden, sodass die Steuereinheit 16 in der Lage ist, anhand dieses Strahlungsbereichs S sicherzustellen, dass eine ordentliche Desinfektion der Oberfläche 30 stattfinden kann. Die Steuereinheit 16 kann nämlich garantieren, dass stets ein maximal wirksamer Abstand zwischen dem Leuchtmittel 18 und der Oberfläche 30, die es zu desinfizieren gilt, eingehalten wird. Zur Unterstützung kann die Detektiereinheit 14 die entsprechende Entfernung dynamisch erfassen und an die Steuereinheit 16 weiterleiten. Eine mögliche oder erforderliche Abstandskorrektur wird dann im Display 13 angezeigt. Eine notwendige Korrektur kann durch Vibration der Griffeinheit 10 oder ein akustisches Signal dem Benutzer mitgeteilt werden, so dass diese umgehend umgesetzt werden kann. In dieser Ausführungsform können einfache Oberflächen, wie zum Beispiel von Geräten, Stühlen oder Tischen mit der genannten optischen Strahlung beaufschlagt werden, um mögliche Krankheitserreger zu verringern oder zu beseitigen.

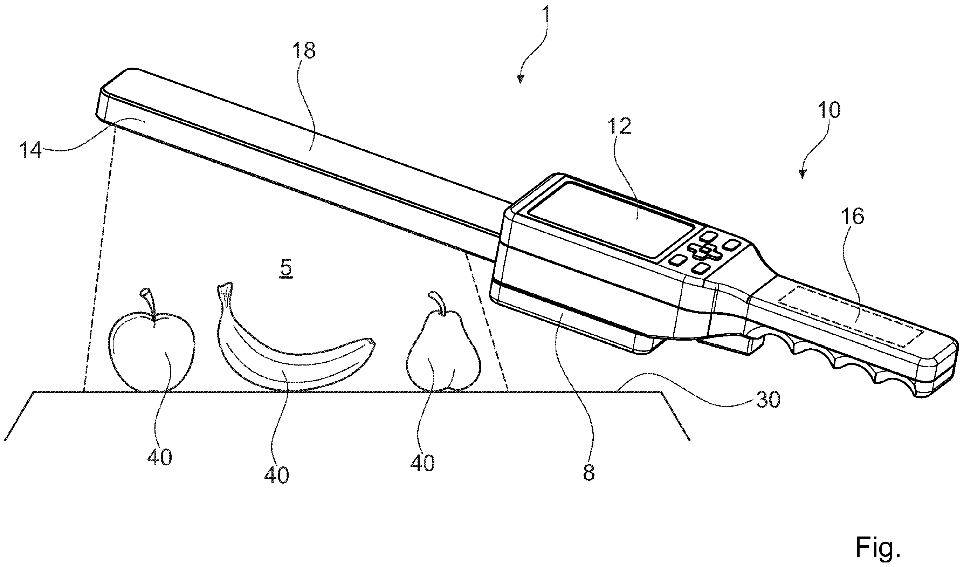

**Fig. 5** zeigt eine weitere Ansicht der erfindungsgemässe Vorrichtung 1 im Einsatz. Hier wird die Vorrichtung 1 in einem Abstand über Objekte 40 gehalten. Es ergibt sich ebenfalls ein entsprechender Strahlungsbereich S. Dieser Strahlungsbereich S wird von der Steuereinheit 16 so eingestellt, dass die Steuereinheit 16 in der Lage ist, anhand des Strahlungsbereichs S sicherzustellen, dass eine ordentliche Desinfektion aller Objekte 40 stattfinden kann.

Die Steuereinheit 16 versucht, stets einen maximal wirksamen Abstand zwischen dem Leuchtmittel 18 und den Objekten 40 einzuhalten. Erforderliche Abstandskorrekturen werden dem Benutzer zur Umsetzung im Display 13 angezeigt.

Sollte ein Benutzer die Vorrichtung 1 in Richtung Körper halten und die Detektiereinheit 14 erkennt beispielsweise Augen, dann wird die Bestrahlung sofort gestoppt bzw. unterbrochen.

Die Detektiereinheit 14 kann auch ausgebildet sein, um eine Distanz zu messen. Zusätzlich können (nicht gezeigt) Accelerometer und Magnetometer vorgesehen sein, welche die Lage des Leuchtmittels 18 im Raum erkennt.

Die Steuereinheit 16, kann z.B. mittels Detektiereinheit 14 und Datensätzen, Geräte oder Gerätetypen erkennen und die Konfiguration der Vorrichtung 1 so anpassen, dass ein entsprechendes Programm sicherstellt, dass die Geräte oder Gerätetypen optimal behandelt werden. Eine Erkennung von Geräten mittels Bluetooth oder RFID ist möglich und von der Vorrichtung 1 verarbeitbar.

Es wurde überraschend festgestellt, dass Wasser der Wirkungsweise des entsprechenden UV-Lichts im genannten Spektrum in keiner Weise abträglich als Übertragungsmedium ist. So kann auch sichergestellt werden, dass Wasser oder ähnliche Flüssigkeiten stets keimfrei oder nahezu keimfrei sind. Die Leuchtmittel 18 können entsprechend der vorangehend beschriebenen Ausführungsformen eine Wellenlänge mit einem Peak im Bereich zwischen 207 bis 222 nm emittieren, wobei eine Wellenlänge gewählt werden kann, oder eine Kombination aus einer Mehrzahl an Wellenlängen und entsprechende Kurzpass- und/oder Bandpassfilter vorgesehen sein können, um den Peak schmal zu halten.

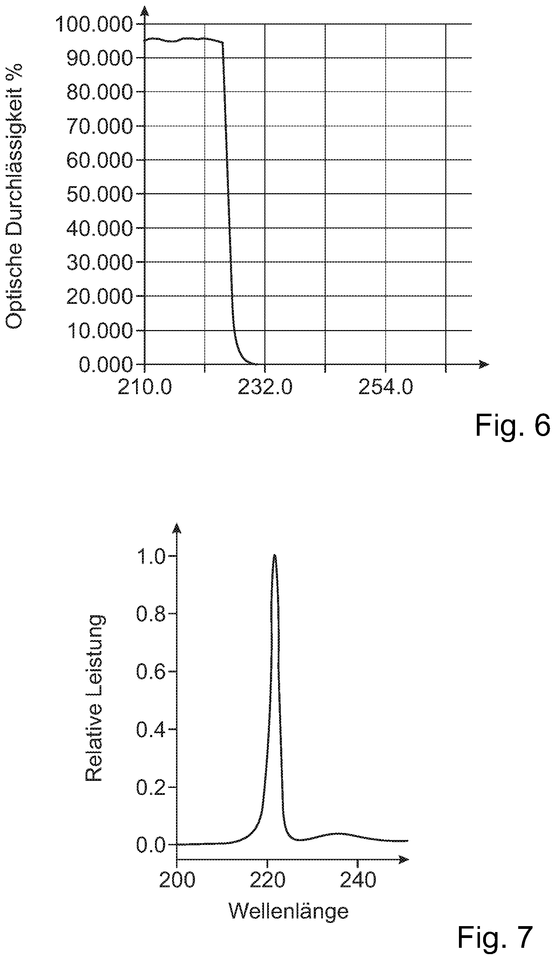

Die **Fig. 6** zeigt eine Transmissionskurve eines geeigneten Filters für ein Leuchtmittel 18, z.B. wie es in der Ausführungsform gemäss der Fig. 1 verwendet wurde. Beim verwendeten Filter handelt es sich um einen Filter auf der Basis eines doppelt beschichteten synthetischen Quarzglases, z.B. aus SiCl₄ (Siliziumtetrachlorid). Die Beschichtung dient als Interferenzfilter und kann mittels eines physikalischen oder chemischen Bedampfungsverfahrens über Gasphasenabscheidung erfolgen. Besonders bevorzugt weisen die verwendeten Filter einen scharfen Übergang zwischen Transmission und Reflexion auf.

Der beispielhaft gezeigte Kurzpassfilter verfügt über eine optische Durchlässigkeit von mehr als 90% im Bereich zwischen 210 und ca. 230 nm, wobei die Kante bei 229 bis 232 nm liegt.

**Fig. 7** zeigt beispielhaft eine erzeugte Wellenlänge für den erfindungsgemässen Gebrauch mit einem Peak im Bereich von 222 nm. Eine solche ist z.B. erzeugbar bei Verwendung einer Krypton-Chlor-Excimer-Lampe mit einem Emissionsspektrum mit einem Peak bei 222 nm, nachdem sie mit einem Filter, wie zur Fig. 6 beschreiben, gefiltert wurde.

Die relative Leistung liegt im Wesentlichen im Bereich von 222 nm, wobei eine Halbwertsbreite des Spektrums von ca. 4 nm im vorliegenden Beispiel vorliegt.

Die vorliegende Erfindung zeigt Vorrichtungen und die Verwendung von Leuchtmitteln, sowie Verfahren zum Betreiben von genannten Vorrichtungen, welche geeignet sind, Krankheitserreger zu vermindern oder gar zu beseitigen. Somit können hygienisch einwandfreie Oberflächen und Bereiche geschaffen werden, welche einer Verbreitung von Keimen entgegenwirken.

Es versteht sich von selbst, dass für einen Fachmann zahlreiche weitere Anwendungsgebiete im Gebiet der Krankheitserregerbeseitigung anhand der exemplarisch beschriebenen Ausführungsbeispiele denkbar sind.

### Liste der verwendeten Referenzeichen

1 tragbare Vorrichtung
8 Energiequelle
10 Griffeinheit
11a Schalter
11b Drehrad
12 Bedienelement
13 Display
14 Detektiereinheit
16 Steuereinheit
18 Leuchtmittel
19 Reflektorschirm
20 Luftkühlung
22 Aufbewahrungsbox
30 Oberfläche
33 Kabel
40 Objekt

## Patentansprüche

1. Tragbare Vorrichtung (1) zur Verminderung oder Beseitigung von Krankheitserregern, umfassend:
a. eine Griffeinheit (10), die mindestens ein Bedienelement (12) umfasst;
b. mindestens ein Leuchtmittel (18), das mit der Griffeinheit (10) verbindbar und dazu ausgelegt ist, optische Strahlung in einem Wellenlängenbereich von zwischen 200 nm und 230 nm abzustrahlen, und wobei das mindestens eine Leuchtmittel ausgelegt ist, einen Strahlungsbereich (S) mit optischer Strahlung mit einem Peak in einem Wellenlängenbereich von zwischen 207 nm und 222 nm zu beaufschlagen;
c. mindestens eine Detektiereinheit (14) zur Ermittlung von Parametern, wobei die Detektiereinheit (14) mit optischen Sensoren zur optischen Erkennung des Bestrahlungsgegenstands ausgestattet ist und
d. eine Steuereinheit (16), die vorzugsweise in der Griffeinheit (10) angeordnet ist, wobei die Steuereinheit (16) das Leuchtmittel (18) mittels Bedienelement (12) und in Abhängigkeit der von der Detektiereinheit (14) ermittelten Parameter steuert.

2. Vorrichtung gemäss Anspruch 1, wobei das Leuchtmittel (18) ausgelegt ist, den Strahlungsbereich (S) mit optischer Strahlung mit einem Peak in einem Wellenlängenbereich von zwischen 207 nm und 222 nm zu beaufschlagen, so dass innerhalb des Strahlungsbereichs eine Dosis von zwischen 0.5 mJ/cm² bis 500 mJ/cm2, insbesondere von zwischen 2 mJ/cm² und 50 mJ/cm², ganz besonders bevorzugt von ca. zwischen 2 mJ/cm² und 20 mJ/cm² vorliegt.

3. Vorrichtung gemäss einem der Ansprüche 1 oder 2, wobei das Leuchtmittel (18) eine Excimer-basierende Lampe umfasst, insbesondere eine Kr-Br-Excimer oder eine Kr-CI-Excimer Lampe umfasst.

4. Vorrichtung gemäss einem der Ansprüche 1 bis 3, wobei das Leuchtmittel (18) einen Bandpassfilter und/oder Kurzpassfilter umfasst, der ausgelegt ist, Wellenlängen innerhalb eines Spektralbereichs von zwischen 200 nm und 230 nm hindurchzulassen.

5. Vorrichtung gemäss Anspruch 4, wobei der Bandpassfilter ein Kurzpassfilter mit eine Transmissionsbereich von weniger als 230 nm ist, insbesondere ein Kurzpassfilter mit einer Kante einem Bereich von zwischen 226 nm und 232 nm ist, insbesondere mit einer Kante einem Bereich einem Bereich von zwischen 229 nm und 232 nm ist.

6. Vorrichtung gemäss einem der Ansprüche 1 bis 5, wobei die Detektiereinheit (14) den Abstand zu einer Oberfläche (30) ermittelt.

7. Vorrichtung gemäss einem der Ansprüche 1 bis 6, wobei die Detektiereinheit (14) die Lage des Leuchtmittels (18) relativ zu einem Objekt (40) erfasst.

8. Vorrichtung gemäss einem der Ansprüche 1 bis 7, wobei die Detektiereinheit (14) eine Bewegung ermittelt.

9. Vorrichtung gemäss einem der Ansprüche 1 bis 8, wobei die Detektiereinheit (14) Objekte erkennt.

10. Vorrichtung gemäss einem der Ansprüche 1 bis 9, wobei das Bedienelement (12) ein Display (13) umfasst, das eine Benutzerführung bereitstellt.

11. Vorrichtung gemäss einem der Ansprüche 1 bis 10, wobei die Detektiereinheit (14) und die Steuereinheit (16) die Handhabung der tragbaren Vorrichtung (1) über einen Zeitraum aufzeichnen.

12. Verfahren zur Verminderung oder Beseitigung von Krankheitserregern, umfassend die Schritte:
a. Bereitstellen einer Vorrichtung (1) gemäss einem der Ansprüche 1 bis 11;
b. Positionieren der Vorrichtung (1), so dass mindestens ein zu behandelnder Bereich sich im Strahlungsbereich (S) befindet
c. Beaufschlagen des Strahlenbereichs mit Strahlung mit einem Peak in einem Wellenlängenbereich von zwischen 207 nm und 222 nm.

13. Verfahren gemäss Anspruch 12 wobei das Positionieren durch das Bedienelement (12) von der Steuereinheit (16) aufgrund einer erfassten Lage des Leuchtmittels (18) unterstützt wird.

14. Verfahren gemäss einem der Ansprüche 12 and 13, wobei das Verfahren weiter den Schritt umfasst: Aufzeichnung der Benutzung der Vorrichtung (1) über einen Zeitraum.

## Claims

1. A portable device (1) for reducing or eliminating pathogens, which comprises:
a. a handle unit (10) comprising at least one operating element (12)
b. at least one illumination means (18), which is connectable to the handle unit (10) and is designed to emit optical radiation within a wavelength range of between 200 nm and 230 nm, and wherein the at least one illumination means is designed to expose an irradiated area (S) to optical radiation having a peak within a wavelength range from 207 nm to 222 nm;
c. at least one detector unit (14) for determining parameters, wherein the detector unit (14) is equipped with optical sensors for optical recognition of the irradiated object, and
d. a control unit (16), which is preferably arranged in the handle unit (10), wherein the control unit (16) controls the illumination means (18) by means of the operating element (12) and depending on the parameters determined by the detector unit (14).

2. The device according to claim 1, wherein the illumination means (18) is designed to expose the irradiated area (S) to optical radiation having a peak within a wavelength range between 207 nm and 222 nm so that a dose of between 0.5 mJ/cm² and 500 mJ/cm², in particular between 2 mJ/cm² and 50 mJ/cm², and quite particularly preferably between approximately 2 mJ/cm² and 20 mJ/cm² is provided.

3. The device according to one of claims 1 or 2, wherein the illumination means (18) comprises an excimer-based lamp, and in particular comprises a Kr-Br excimer lamp or a Kr-Cl excimer lamp.

4. The device according to one of claims 1 to 3, wherein the illumination means (18) comprises a bandpass filter and/or a shortpass filter, which is designed permit passage of wavelengths within a spectral range between 200 nm and 230 nm.

5. The device according to claim 4, wherein the bandpass filter is a shortpass filter having a transmission range of less than 230 nm, in particular a shortpass filter having a cutoff wavelength in a range between 226 nm and 232 nm, in particular having a cutoff wavelength in a range between 229 nm and 232 nm.

6. The device according to one of claims 1 to 5, wherein the detector unit (14) determines the distance to a surface (30).

7. The device according to one of claims 1 to 6, wherein the detector unit (14) recognizes the location of the illumination means (18) in relation to an object (40).

8. The device according to one of claims 1 to 7, wherein the detector unit (14) determines a movement.

9. The device according to one of claims 1 to 8, wherein the detector unit (14) recognizes objects.

10. The device according to one of claims 1 to 9, wherein the operating element (12) comprises a display (13) which provides guidance to the user.

11. The device according to one of claims 1 to 10, wherein the detector unit (14) and the control unit (16) record the handling of the portable device (1) during a chronological period.

12. A method for reducing or eliminating pathogens, which comprises the following steps:
a. providing a device (1) according to one of claims 1 to 11;
b. positioning the device (1) so that at least one area being treated is located in the irradiated area (S);
c. exposing the irradiated area to radiation having a peak within a wavelength range between 207 nm and 222 nm.

13. The method according to claim 12, wherein said positioning is assisted by the operating element (12) of the control unit (16) on the basis of the detected location of the illumination means (18).

14. The method according to one of claims 12 and 13, wherein the method further comprises the following step: recording the usage of the device (1) during a chronological period.

## Revendications

1. Dispositif portable (1) pour réduire ou éliminer des agents pathogènes, comprenant :
a. une unité de poignée (10) qui comprend au moins un élément de commande (12) ;
b. au moins une source lumineuse (18), qui peut être reliée à l'unité de poignée (10) et est conçue pour émettre un rayonnement optique dans une plage de longueurs d'onde comprise entre 200 nm et 230 nm, et où l'au moins une source lumineuse est conçue pour exposer une zone de rayonnement (S) avec un rayonnement optique ayant un pic dans une plage de longueurs d'onde comprise entre 207 nm et 222 nm ;
c. au moins une unité de détection (14) pour déterminer des paramètres, où l'unité de détection (14) est équipée de capteurs optiques pour la reconnaissance optique de l'objet à irradier et
d. une unité de commande (16), qui est agencée de préférence dans l'unité de poignée (10), où l'unité de commande (16) commande la source lumineuse (18) au moyen d'un élément de commande (12) et en fonction des paramètres déterminés par l'unité de détection (14).

2. Dispositif selon la revendication 1, dans lequel la source lumineuse (18) est conçue pour exposer la zone de rayonnement (S) avec un rayonnement optique ayant un pic dans une plage de longueurs d'onde comprise entre 207 nm et 222 nm, de sorte que, à l'intérieur de la zone de rayonnement, il y ait une dose comprise entre 0,5 mJ/cm² à 500 mJ/cm², en particulier entre 2 mJ/cm² et 50 mJ/cm², de manière tout particulièrement préférée entre environ 2 mJ/cm² et 20 mJ/cm².

3. Dispositif selon l'une quelconque des revendications 1 ou 2, dans lequel la source lumineuse (18) comprend une lampe à base d'excimère, en particulier comprend une lampe excimère Kr-Br ou une lampe excimère Kr-CI.

4. Dispositif selon l'une quelconque des revendications 1 à 3, dans lequel la source lumineuse (18) comprend un filtre passe-bande et/ou un filtre passe-bas, qui est conçu pour laisser passer des longueurs d'onde comprises dans une plage spectrale comprise entre 200 nm et 230 nm.

5. Dispositif selon la revendication 4, dans lequel le filtre passe-bande est un filtre passe-bas avec une plage de transmission inférieure à 230 nm, en particulier un filtre passe-bas avec un flanc dans une plage comprise entre 226 nm et 232 nm, en particulier avec un flanc dans une plage comprise entre 229 nm et 232 nm.

6. Dispositif selon l'une des revendications 1 à 5, dans lequel l'unité de détection (14) détermine la distance par rapport à une surface (30).

7. Dispositif selon l'une des revendications 1 à 6, dans lequel l'unité de détection (14) détecte la position de la source lumineuse (18) par rapport à un objet (40).

8. Dispositif selon l'une des revendications 1 à 7, dans lequel l'unité de détection (14) détecte un mouvement.

9. Dispositif selon l'une des revendications 1 à 8, dans lequel l'unité de détection (14) reconnaît des objets.

10. Dispositif selon l'une des revendications 1 à 9, dans lequel l'élément de commande (12) comprend un affichage (13) qui fournit un guide utilisateur.

11. Dispositif selon l'une des revendications 1 à 10, dans lequel l'unité de détection (14) et l'unité de commande (16) enregistrent la manipulation du dispositif portable (1) sur une période de temps.

12. Procédé pour réduire ou éliminer des agents pathogènes, comprenant les étapes de
a. fourniture d'un dispositif (1) selon l'une quelconque des revendications 1 à 11;
b. positionnement du dispositif (1) de manière à ce qu'au moins une zone à traiter se trouve dans la zone de rayonnement (S);
c. exposition de la zone de rayonnement avec un rayonnement ayant un pic dans une plage de longueurs d'onde comprise entre 207 nm et 222 nm.

13. Procédé selon la revendication 12, dans lequel le positionnement par l'élément de commande (12) est pris en charge par l'unité de commande (16) sur la base d'une position détectée de la source lumineuse (18).

14. Procédé selon l'une des revendications 12 ou 13, le procédé comprenant en outre l'étape de : enregistrement de l'utilisation du dispositif (1) sur une période de temps.
